# EUROPEAN PATENT APPLICATION

(11) **EP 2 157 072 A1**
(43) Date of publication of application: **24.02.2010**
(21) Application number: 08753915.1
(22) Date of filing: 11.04.2008
(51) Int. Cl.: C07C 11/18, C07C 1/20

(54) **ISOPRENE PRODUCTION METHOD**

(30) Priority: 03.05.2007 RU 2007116797
(71) Applicant: Obshestvo S Ogranichennoi Otvetstvennoctiu 'Eurochim-SPB-Trading', St.Petersburg 190020 (RU)
(72) Inventor: BUSYGIN, Vladimir Mikhailovich, Nizhnekamsk, 423550 (RU); DYKMAN, Arkady Samuilovich, St.Petersburg, 194356 (RU); GILMANOV, Khamit Khamisovich, Nizhnekamsk, 423570 (RU); TULCHINSKY, Eduard Abramovich, Nizhnekamsk, 423550 (RU); POLYAKOV, Sergey Aleksandrovich, St.Petersburg, 192177 (RU)
(74) Representative: Henrion, Oliver
(86) International application number: PCT/RU2008/000225
(87) International publication number: WO 2008/136706

(57) **Abstract**

This invention is applicable in the area of basic-type organic and petrochemical synthesis - specifically, to methods applicable to obtaining of isoprene out of isobutylene and formaldehyde - and can be used in manufacture of artificial resins.

The proposed method is designed to obtain isoprene by way of liquid-phase interaction between trimethyl carbinol (or its water solutions) and formaldehyde (or its source substances) in the presence of acidic catalyzer water solution; this can be made in one or several contacting stages, with use (at the final contacting stage) of separation reactor containing a heat supply zone, a reaction zone and a separation zone, with reaction products and water taken, out of the separation zone, in the form of a vapor flow to be subsequently cooled down, condensed and separated and with liquid flow of the catalyzer water solution put out for extraction and, after this, put back into the heating zone. As it goes from the reaction zone into the separation zone, the reactive flow is throttled. In the reaction zone, temperature is maintained at the level of 140-180 °C, while pressure is 8-25 atmospheres; in the separation zone, pressure is 1.2-9.5 atmospheres. The separation reactor contains two or three separation zones. The balance quantity of water is put out of catalyzer water solution, which is circulating along the circuit, by way of its evaporation as the flow is throttled into the separation zone (zones) during regulation of the quantity of the circulating liquid phase in the interval of 0.2-6.0 parts of the total reaction zone area.

## Description

This invention is a process for the preparation of isoprene out of isobutylene and formaldehyde.

Isoprene is widely usable as a monomer for obtaining of rubbers, such as are similar in their properties to natural ones, and in organic synthesis.

There are a number of methods known as serving to obtain isoprene through condensation of isobutylene and formaldehyde or their source substances, such as 4,4-dimethyl-1,3-dioxane and trimethyl carbinol, in the presence of acidic catalyzer water solution, which can be made under a higher temperature and a higher pressure, in one or several stages of contacting, with distillation, on the final stage of the contacting, of the easy-boiling part of reaction products and water in separation reactors of various design, which usually include a heat supply zone, a reaction zone, a separation zone and a circulation circuit [Russian Patent 1216940, Russian Patent 2085552, Russian Patent 2098398, Russian Patent 2099318, Russian Patent 2203878, Russian Patent 2280022, US Patent 4511751, EP Patent 0106323].

The known methods for obtaining of isoprene, in order to keep amount of the catalyzer solution constant, requires distillation of the balance quantity of water produced by the reaction and put in together with the reagents. Furthermore, high-boiling by-products have also to be (continuously or from time to time) put out of the catalyzer solution.

Method essentially most similar to the herein proposed invention is described in Patent EP 0106323. What is stated in that patent is maintaining of conditions required for the process within such interval, as is ensuring distillation of the yielded isoprene, easy-boiling source products and by-products and the balance quantity of water. Specifically, the following conditions of the process are stated: temperature equal to 150-220°C, pressure 1.1-2.5 times higher than pressure of saturated water vapors over acidic catalyzer solution, temperature: that of the process, molar ratio of isobutylene (and/or the source of isobutylene) and formaldehyde (and/or the source of formaldehyde): not less than 3.

Under the known method, ratio between the distillable easy-boiling products and water can be controlled by way of pressure regulation in the reaction system. If pressure in the reaction system grows higher, ratio between water and other components of the distillate becomes lower, while a lowering of pressure maintained in the reaction system has the opposite effects.

Essential disadvantage of the known technology is that parameters of the process are shaped by the necessity to provide distillation of the balance water quantity, and not by conditions optimal for the reaction.

For instance, the reaction is more selective under a lower temperature, but, in such a case, there is no balance water quantity distillation, the catalyzer solution gets diluted with water, its acidity falls down to lesser levels, and conversion rates of the reagents and yield outputs of the reaction volume are also lower. Therefore, the only way left is either an increase of the temperature and having to operate with the inevitably lower selectivity levels or a reduction of pressure, which also produces a lower selectivity.

It is known that selectivity of the process depends on isobutylene concentration (solubility) in reactive water phase, which, in its own turn, depends on partial pressure of isobutylene in the reaction zone. For this reason, a relatively lower pressure level required in the reaction zone for provision of the balance water quantity reduces selectivity of the process.

It is especially important when source material of the process is trimethyl carbinol (the source of isobutylene) or any of its water solutions (water azeotrope containing 12-13% of water, usually) yielded by its production through hydration of isobutylene within C₄ fractions. This requires output of a higher quantity of water produced by hydration of trimethyl carbinol and put in together with it.

A simple calculation shows that, if the process is maintained at 160°C and relies upon 40% formaldehyde and 88% trimethyl carbinol (all the rest being water) as source materials, maintaining of distillation of the balance water quantity, with mole ratio of the reagents making 1:10, requires pressure to be maintained in the reaction zone at the level of not more than 9.1 atmospheres or, if mole ratio of the reagents is 1:5, not more than 8.6 atmospheres.

Similarly, if the source materials are 4,4-dimethyl-1,3-dioxane and 88% trimethyl carbinol, with mole ratio of the reagents equal to 1:10, reaction zone pressure should be not more than 9.7 atmospheres or, if mole ratio of the reagents is 1:5, not more than 9.6 atmospheres.

Essential purpose of this invention is removal of the above stated disadvantages.

The purpose was achieved through separation of the reaction zone and separation zone and through the shaping of conditions optimal for each zone. As the reactive mixture was moving from the reaction zone to the separation zone, the flow was going through its throttling.

Throttling of the flow is a process involving higher water evaporation rates dependent on the essential nature and concentrations of substances present there and on the initial and final parameters of the throttling.

According to our findings, there are, for each specific reactive mixture, specific parameters required to maintain the necessary water evaporation rate; intervals in the values of such parameters are identified.

We are proposing, herein, an isoprene obtaining method, which makes it possible to avoid having to keep reaction zone pressure bound to water vapor partial pressure rates corresponding to reaction zone temperature (and causing distillation of the balance water quantity) and to maintain isoprene synthesis reaction in conditions optimal for it in terms of temperature, pressure and reagent ratios. Here, putting of the balance water quantity out of the catalyzer solution is maintainable through regulation of separation zone parameters and regulation of circulating solution quantity.

We are proposing a method for obtaining of isoprene through liquid-phase interaction between trimethyl carbinol or its water solutions and formaldehyde or its source substances, such as 4,4-dimethyl-1,3-dioxane, in the presence of acidic catalyzer water solution, maintainable under a higher temperature and pressure, in one or several contacting stages, with use (at the final contacting stage) of a separation reactor containing a heat supply zone, a reaction zone and a separation zone, with removal of reaction products and water out of the separation zone in the form of a vapor flow, with the subsequent cooling, condensation and separation, with removal of catalyzer water solution liquid flow for extraction, with the subsequent return of the staff into the heating zone, where the maintained reaction zone pressure is higher than that of saturated water vapor pressure corresponding to temperature maintained in the reaction zone, while the maintained separation zone pressure is lower than that of saturated water vapor pressure corresponding to reaction zone temperature, with the reactive flow throttled during its transition from the reaction zone to the separation zone. For this purpose, the reaction zone is operated under 140-180°C and 8-25-atmosphere pressure, while pressure maintained in the separation zone is equal to 1.2-12 atmospheres.

In correspondence with the proposed invention, the usable separation reactor contains a heat supply zone, a contacting zone and a separation zone, each of which can be designed as a part of an integrated whole or can make up an individual unit of the equipment.

Throttling of the flow out of the reaction zone into the separation zone can be maintained with use of pressure regulator valve or by any other methods available for creation of a difference in pressure levels between the zones (installation of a shut-off & control valve system, small-section pipe lines designed to produce resistance to the flow, etc.).

Heat can be supplied into the reaction zone through use of either heat-exchange internal surface in the zone or external heat exchangers and circulation of the reactive mass through them.

The stirring of the reactive mass in the reaction zone can be maintained with use of mechanical or natural movement of the flows (mixers, circulation pumps, natural movement of the flows maintained by difference in density rates or difference in the levels), by way of fittings-on and mass-exchange equipment installed for the purpose or by any other ways and methods available.

The used separation reactor can contain two or three successive separation zones for preliminary separation of phases and for subsequent selective throttling of one of the phases. Working parameters of the successive separation zones will be naturally dissimilar.

If two separation zones are present the stream leaving the reaction zone is fed into the first separation while the pressure is decreased by 2 to 20 atm. A steam stream leving the first separation zone is subjected to cooling and condensation, whereas a liquid stream is fed into the second separation zone while the pressure is decreased to 1.2 -2.0 atm. A vapour phase is withdrawn and subjected to cooling and condensation. A liquid phase so obtained is withdrawn, cooled, extracted and recyled into a heating zone.

If three separation zones are present the first zone is used for a preliminary more efficient phase separation at a temperature and pressure that are near to temperature and pressure in the reaction zone.

When conducting the present process the removal of balance water amount from the aqueous catalyst solution (reactive liquid phase) that is circulated in the following loop: reaction zone-separation zone-extraction zone-heating zone-reaction zone may be additionally regulated by varying the amount of circulating liquid phase. When increasing circulation the stream of liquid phase fed to the separaration zone(s) is increased and the amount of additionally evaporating water is increased, respectively. On the other hand when decreasing circulation the amount of additionally evaporating water is decreased. The amount of circulating liquid phase may be varied in the range of 0.2-6.0 parts per volume of the reaction zone.

The proposed isoprene obtaining method not only makes it possible to create, in the reaction zone, a set of conditions optimal for chemical reactions to be maintained, but also offers a number of other advantages.

It is known that reactive water phase (water solution of the acid) is, in conditions of the ongoing process (a high temperature), highly active in terms of corrosion, for reason of which the usable separation reactor should be made of highly alloyed materials resistant against corrosion. Within method proposed by us herein, separation zone working parameters are lower in temperature and pressure, which largely cuts the usual corrosion rates of materials usable and makes it possible to make a part of the equipment (the separation zone) of such materials, as are alloyed to lesser degrees and have lower metal intensity rates.

A condition indispensable for maintaining of the process is making of the catalyzer solution free of any high-boiling by-products; this is usually made by way of extraction with use of solvent fitting for the purpose.

It is known that high-boiling by-products (resins) produced during the process tend to clog up the in-use technological equipment and pipelines - especially during cooling.

Having investigated the process in correspondence with the proposed invention, we found that, during throttling of the reactive water phase into the separation zone, it cools down by way of water evaporation (by way of boiling-up taking place in case of a pressure reduction down to below the pressure of saturated vapors). The cooling, if made in the boiling-up mode, promotes dispersion of resins' hetero phase present at it and prevents its peeling-off and clogging of the equipment during further processing of the acid solution.

The essential distinctive feature of the proposed method is that the process takes place in separation reactor, where the maintained reaction zone pressure is higher than saturated water vapor pressure corresponding to reaction zone temperature, while the maintained separation zone pressure is lower than saturated water vapor pressure corresponding to reaction zone temperature and throttles the reactive flow during its transit from the reaction zone to the separation zone. For this purpose, regulation of balance water quantity removal is maintained through regulation of separation zone pressure and quantity of the catalyzer's circulating water solution.

This method makes it possible to maintain the process continuously, with the balance water quantity put out, in the form of a vapor flow, along with reaction products and, at the same time, to support, in the reaction zone, optimal temperature and pressure at such levels, as are not involving the necessity to put out the balance water quantity.

Industrial feasibility of the method is demonstrated at pictures of Examples 1-5, while Examples 6 and 7 are shown for reference only.

### Example 1

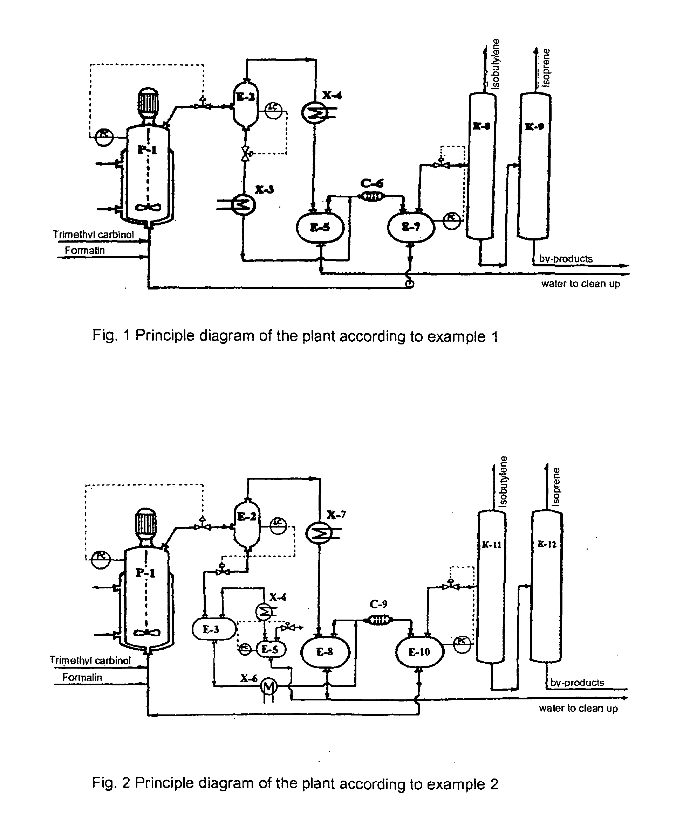

Layout of equipment for use of the method according to the proposed invention is shown at Fig.1.

The equipment contains Reactor 1, Separator 2, Refrigerator 3, Condensing Refrigerator 4, Settler 5, Mixer 6, Settler 7, Isobutylene Distillation Column 8 and Isoprene Distillation Column 9.

Reactor 1, which is a 5-liter autoclave having a mixer provided with an air-tight electro-magnetic drive and a thermal jacket, is fed with 4.8 liters of 7% ortho-phosphoric acid water solution, and, after the stirring is launched (put into the ON mode), hot heat-transferring medium is supplied into the thermal jacket, and the reactor is heated up to 160°C. 40% formaline (expenditure: 256.6 g/hour) and 88% trimethyl carbinol (expenditure: 2875.9 g/hour) are fed into the reactor. Pressure maintained in the reactor is equal to 12.0 atmospheres.

Vapor & liquid flow going out of the reactor is throttled, through PC pressure regulator valve, into Separator 2, where pressure is maintained at the level of 4.5 atmospheres. In Separator 2, the flow is separated into a liquid phase and a vapor phase.

Vapors of reaction products and non-transformed source material vapors, having gone out of Separator 2, go through Condensing Refrigerator 4, where they get cooled and condensed, and proceed to Settler 5 to be split there into a water layer and an organic layer.

The flow of liquid products, which is a mixture of ortho-phosphoric acid water solution and high-boiling by-products, is put out of Separator 2 in the quantity of 30.0 l/hour, cooled down in Refrigerator 3 and supplied to Mixer 6 for extraction of organic products. As extracting agent, the organic layer of synthesis products is supplied into Mixer 6 out of Settler 5. Mixture going out of Mixer 6 enters Settler 7 to be split there into organic layer, containing the extracted high-boiling by-products, and water solution of ortho-phosphoric acid.

Made free of any high-boiling organic products, ortho-phosphoric acid water solution is, with use of the pump, put back into Reactor 1 out of Settler 7.

The organic layer is put out of Settler 7 for separation. Initially, in Rectifying Column 8, isobutylene is isolated in the quantity of 1511.8 g/hour and sent for hydration into trimethyl carbinol (the hydration unit is not shown in the layout illustration); after this, in Rectifying Column 9, isoprene is isolated in the quantity of 162.4 g/hour and delivered for purification.

Column 9 bottom product (in the quantity of 283.0 g/hour), which is a mixture of synthesis by-products and non-transformed source materials, and which contains 78.2% of trimethyl carbinol, 4.1% of methyl dihydropyrane, 0.3% of dimethyl dioxane, 1.0% of isoamylene alcohols and 16.4% of other products, is supplied into equipment unit usable for isolation of non-transformed source materials and interim synthesis products.

Condensed synthesis products' water layer (quantity: 1175.3 g/hour) containing 6.4% of trimethyl carbinol, 0.4% of formaldehyde and 0.2% of other organic products is taken out of Settler 5 and sent for purification before discharge into the chemically polluted sewage system (into the unit for distillation of easy-boiling organic products and formaldehyde).

According to the process balance, isoprene output, in terms of transformed source materials, is: 73.2 mole % for formaldehyde and 74.8 mole % for trimethyl carbinol (isobutylene).

The process is maintained continuously for 40 hours. Per-substance assay of flows sampled after 8, 20 and 40 hours of operation shows identical results, which evidences absence of any water accumulated in the reactive water layer and stability of parameters of the process.

### Example 2

Layout of equipment for use of the method according to the proposed invention is shown at Fig.2.

The equipment contains Reactor 1, Separator 2, Separator 3, Condensing Refrigerator 4, Container 5, Refrigerator 6, Condensing Refrigerator 7, Settler 8, Mixer 9, Settler 10, Isobutylene Distillation Column 11 and Isoprene Distillation Column 12.

Reactor 1, which is a 5-liter autoclave having a mixer provided with an air-tight electro-magnetic drive and a thermal jacket, is fed with 4.8 liters of 3% ortho-phosphoric acid water solution, and, with the stirring in the ON mode, hot heat-transferring medium is supplied into the thermal jacket, and the reactor is heated up to 180°C. 40% formaline (expenditure: 256.6 g/hour) and 88% trimethyl carbinol (expenditure: 2875.9 g/hour) are fed into the reactor. Pressure maintained in the reactor is equal to 25.0 atmospheres.

Vapor & liquid flow going out of the reactor is throttled, through PC pressure regulator valve, into Separator 2, where pressure is maintained at the level of 9.0 atmospheres. In Separator 2, the flow is separated into a liquid phase and a vapor phase.

Vapors of reaction products and non-transformed source material vapors, having gone out of Separator 2, go through Condensing Refrigerator 7, where they get cooled and condensed, and proceed to Settler 8 to be split there into a water layer and an organic layer.

The flow of liquid products, which is a mixture of ortho-phosphoric acid water solution and high-boiling by-products, is put out of Separator 2 through LC-level regulator valve and throttled into Separator 3, where pressure is kept at the level of 2.0 atmospheres. In Separator 3, the liquid phase reaches its ebullience accompanied with a partial steaming-off of water and organic products. Water vapors and vapors of the products go through Condensing Refrigerator 4 to be cooled-down and condensed there and, after this, flow off into Container 5.

The mixture of ortho-phosphoric acid water solution and high-boiling by-products is taken out of Separator 3 and, by way of the pump, delivered (in the quantity of 8.0 l/hour) through Refrigerator 6 into Mixer 9 for extraction of organic products.

As the extracting agent, the organic layer of synthesis products is supplied into Mixer 9 out of Settler 8. Mixture going out of Mixer 9 enters Settler 10 to be split there into organic layer, containing the extracted high-boiling by-products, and ortho-phosphoric acid water solution.

Made free of any high-boiling organic products, ortho-phosphoric acid water solution is, with use of the pump, put back into Reactor 1 out of Settler 10.

The organic layer is put out of Settler 10 for separation. Initially, in Rectifying Column 11, isobutylene is isolated in the quantity of 1555.3 g/hour and sent for hydration into trimethyl carbinol (the hydration unit is not shown in the layout illustration); after this, in Rectifying Column 12, isoprene is isolated in the quantity of 158.3 g/hour and delivered for purification.

Column 12 bottom product (in the quantity of 244.4 g/hour), which is a mixture of synthesis by-products and non-transformed source materials, and which contains 73.9% of trimethyl carbinol, 4.9% of methyl dihydropyrane, 0.4% of dimethyl dioxane, 1.2% of isoamylene alcohols and 19.6% of other products, is supplied into equipment unit usable for isolation of non-transformed source materials and interim synthesis products.

Condensed synthesis products' water layer (in total, in the quantity of 1174.5 g/hour) containing 5.1% of trimethyl carbinol, 0.5% of formaldehyde and 0.3% of other organic products is taken out of Settler 8 and Container 5 and moved out for purification before discharge into the chemically polluted sewage system (into the unit for distillation of easy-boiling organic products and formaldehyde).

According to the process balance, isoprene output, in terms of transformed source materials, is: 71.9 mole % for formaldehyde and 73.4 mole % for trimethyl carbinol (isobutylene).

The process is maintained continuously for 40 hours. Per-substance assay of flows sampled after 8, 20 and 40 hours of operation shows identical results, which evidences absence of any water accumulated in the reactive water layer and stability of parameters of the process.

### Example 3

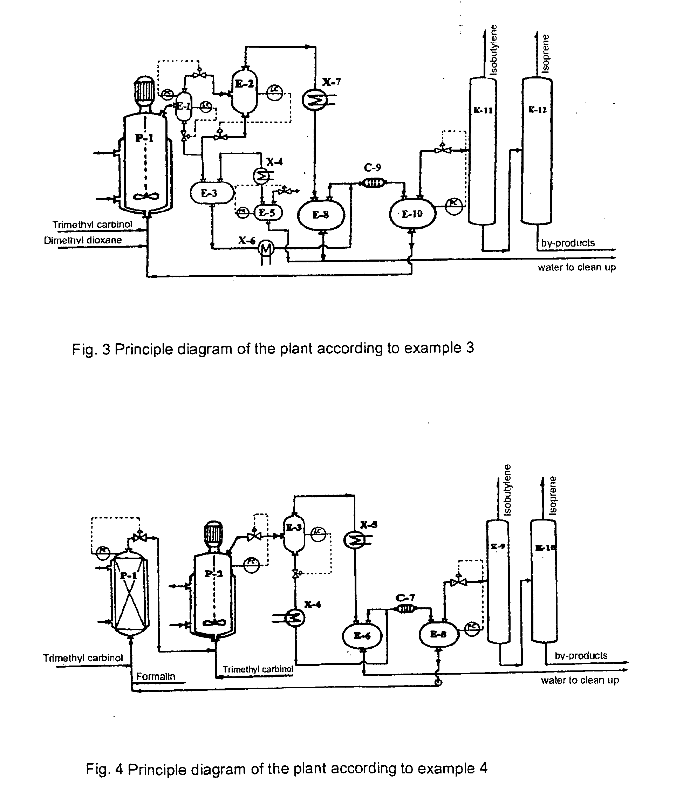

Layout of equipment for use of the method according to the proposed invention is shown at Fig.3.

The equipment contains Reactor 1, Separator 2, Separator 3, Separator 4, Condensing Refrigerator 5, Container 6, Refrigerator 7, Condensing Refrigerator 8, Settler 9, Mixer 10, Settler 11, Isobutylene Distillation Column 12 and Isoprene Distillation Column 13.

Reactor 1, which is a 5-liter autoclave having a mixer provided with an air-tight electro-magnetic drive and a thermal jacket, is fed with 4.8 liters of 9% ortho-phosphoric acid water solution, and, with the stirring in the ON mode, hot heat-transferring medium is supplied into the thermal jacket, and the reactor is heated up to 155°C.

4,4-dimethyl-1,3-dioxane (expenditure: 400.0 g/hour) and 88% trimethyl carbinol (expenditure: 1739.7 g/hour) are fed into the reactor. Pressure maintained in the reactor: 12.0 atmospheres.

Vapor & liquid flow going out of the reactor enters Separator 2, where it is separated into a liquid phase and a vapor phase.

Vapors of reaction products and non-transformed source material vapors, as well as a part of non-separated liquid products (droplet entrainment), having gone out of Separator 2, are throttled, through the pressure regulator valve, into Separator 3, where the maintained pressure is 5.3 atmospheres.

In Separator 3, the flow goes through its further separation into the liquid phase and the vapor phase. Vapors of the products, having gone out of Separator 3, go through Condensing Refrigerator 8, where they get cooled down and condensed, and proceed to Settler 9 to be separated there into a water layer and an organic layer.

The flows of liquid products, which are made up of a mixture of ortho-phosphoric acid water solution and high-boiling by-products, are put out of Separator 2 and Separator 3 through LC-level regulator valves and throttled into Separator 4, where pressure is kept at the level of 1.2 atmospheres. In Separator 4, the liquid phase reaches its ebullience accompanied with a partial steaming-off of water and organic products. Water vapors and vapors of the products go through Condensing Refrigerator 5 to be cooled-down and condensed there and, after this, flow off into Container 6.

The mixture of ortho-phosphoric acid water solution and high-boiling by-products is taken out of Separator 4 and, by way of the pump, delivered (in the quantity of 1.0 l/hour) through Refrigerator 7 into Mixer 10 for extraction of organic products.

As the extracting agent, the organic layer of synthesis products is supplied into Mixer 10 out of Settler 9. Mixture going out of Mixer 10 enters Settler 11 to be split there into organic layer, containing the extracted high-boiling by-products, and ortho-phosphoric acid water solution.

Made free of any high-boiling organic products, ortho-phosphoric acid water solution is, with use of the pump, put back into Reactor 1 out of Settler 11.

The organic layer is put out of Settler 11 for separation. Initially, in Rectifying Column 12, isobutylene is isolated in the quantity of 810.5 g/hour and sent for hydration into trimethyl carbinol (the hydration unit is not shown in the layout illustration); after this, in Rectifying Column 13, isoprene is isolated in the quantity of 346.6 g/hour and delivered for purification.

Column 13 bottom product (in the quantity of 291.4 g/hour), which is a mixture of synthesis by-products and non-transformed source materials, and which contains 59.1% of trimethyl carbinol, 7.7% of methyl dihydropyrane, 2.9% of dimethyl dioxane, 2.2% of isoamylene alcohols and 28.1% of other products, is supplied into equipment unit usable for isolation of non-transformed source materials and interim synthesis products.

Condensed synthesis products' water layer (in total, in the quantity of 691.2 g/hour) containing 8.3% of trimethyl carbinol, 0.3% of formaldehyde and 0.2% of other organic products is taken out of Settler 9 and Container 6 and sent for purification before discharge into the chemically polluted sewage system (into the unit for distillation of easy-boiling organic products and formaldehyde).

According to the process balance, isoprene output is as follows: 151.0 mole % for dimethyl dioxane, which makes 75.5% of the expected output, and 163.6 mole % for trimethyl carbinol (isobutylene), which makes 81.8% of the expected output.

The process is maintained continuously for 40 hours. Per-substance assay of flows sampled after 8, 20 and 40 hours of operation shows identical results, which evidences absence of any water accumulated in the reactive water layer and stability of parameters of the process.

### Example 4

Layout of equipment for use of the method according to the proposed invention is shown at Fig.4.

The equipment contains Reactors 1 and 2, Separator 3, Refrigerator 4, Condensing Refrigerator 5, Settler 6, Mixer 7, Settler 8, Isobutylene Distillation Column 9 and Isoprene Distillation Column 10.

To maintain the process in two contacting stages, the reactor unit is made up of Reactor 2 (for synthesis of isoprene's predecessors) and Reactor 1 (for decomposition of isoprene's predecessors into isoprene).

Reactor 1 is a vertical column-type device (height: 3 meters, diameter: 0.05 meters) filled in with the fitting-on and having a thermal jacket. Reactor 2 is a 5-liter autoclave having a mixer provided with an air-tight electro-magnetic drive and a thermal jacket.

The reactor unit is fed with 9.0 liters of 7% ortho-phosphoric acid water solution, hot heat-transferring medium is supplied into the thermal jackets, Reactor 2 is heated up to 110°C, and Reactor 1 (with the stirring in the ON mode) is heated up to 160°C.

Reactor 1 is fed into with 40% formaline (expenditure: 256.6 g/hour) and 88% trimethyl carbinol (expenditure: 860.0 g/hour), as well as with circulation solution of the acid out of Container E-8 (expenditure: 3.0 l/hour). Pressure maintained in the reactor: 22.0 atmospheres.

Flow going out of Reactor 1 is mixed with a further quantity of trimethyl carbinol supplied in the amount of 2015.9 g/hour and delivered into Reactor 2.

Pressure maintained in Reactor 2: 12.0 atmospheres.

Vapor & liquid flow going out of the reactor is throttled, through PC pressure regulator valve, into Separator 3, where pressure is maintained on the level of 4.5 atmospheres. In Separator 3, the flow is separated into a liquid phase and a vapor phase.

Vapors of reaction products and non-transformed source material vapors, having gone out of Separator 3, go through Condensing Refrigerator 5, where they are cooled-down and condensed, and enter Settler 6, where they are split into a water layer and an organic layer.

The flow of liquid products, which is a mixture of ortho-phosphoric acid water solution and high-boiling by-products, are put out of Separator 3 in the quantity of 30.0 l/hour, cooled down in Refrigerator 4 and delivered into Mixer 7 for extraction of organic products. As the extracting agent, organic layer of synthesis products is supplied into Mixer 7 out of Settler 6. Mixture going out of Mixer 7 enters Settler 8, where it is split into organic layer, containing the extracted high-boiling by-products, and water solution of ortho-phosphoric acid.

Made free of any high-boiling organic products, ortho-phosphoric acid water solution is, with use of the pump, put back into Reactor 1 (in the quantity of 3.0 l/hour) and into Reactor 2 (in the quantity of 27.0 l/hour).

The organic layer is put out of Settler 8 for separation. Initially, in Rectifying Column 9, isobutylene is isolated in the quantity of 1515.5 g/hour and sent for hydration into trimethyl carbinol (the hydration unit is not shown in the layout illustration); after this, in Rectifying Column 10, isoprene is isolated in the quantity of 164.4 g/hour and delivered for purification.

Column 10 bottom product (in the quantity of 273.1 g/hour), which is a mixture of synthesis by-products and non-transformed source materials, and which contains 84.0% of trimethyl carbinol, 4.4% of methyl dihydropyrane, 0.5% of dimethyl dioxane, 0.9% of isoamylene alcohols and 10.2% of other products, is supplied into equipment unit usable for isolation of non-transformed source materials and interim synthesis products.

Condensed synthesis products' water layer (in total, in the quantity of 1179.5 g/hour) containing 6.9% of trimethyl carbinol, 0.5% of formaldehyde and 0.2% of other organic products is taken out of Settler 6 and sent for purification (into the unit for distillation of easy-boiling organic products and formaldehyde) before discharge into the chemically polluted sewage system.

According to the process balance, isoprene output, in terms of transformed source materials, is: 74.9 mole % for formaldehyde and 82.3 mole % for isobutylene.

The process is maintained continuously for 40 hours. Per-substance assay of flows sampled after 8, 20 and 40 hours of operation shows identical results, which evidences absence of any water accumulated in the reactive water layer and stability of parameters of the process.

### Example 5

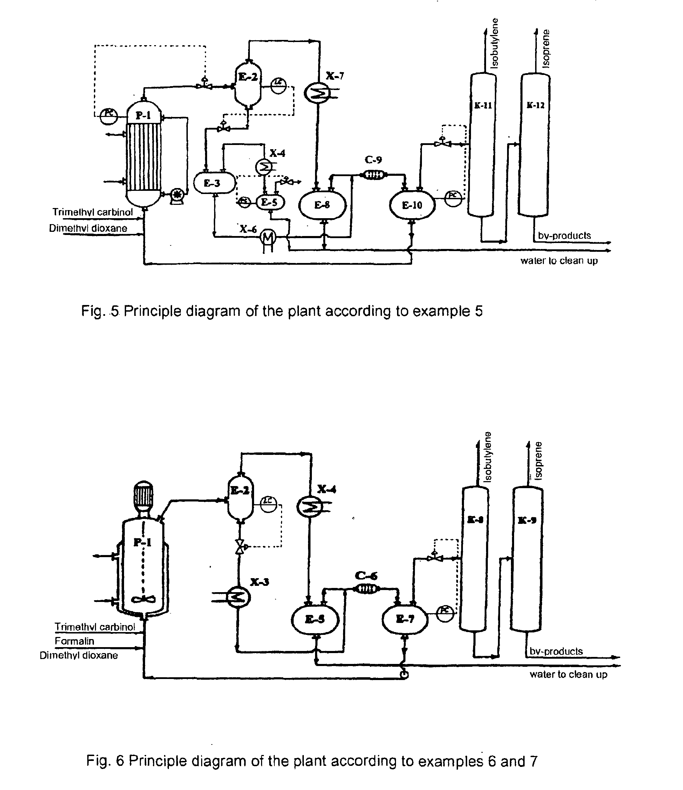

Layout of equipment for use of the method according to the proposed invention is shown at Fig.5.

The equipment contains Reactor 1, Separator 2, Separator 3, Condensing Refrigerator 4, Container 5, Refrigerator 6, Condensing Refrigerator 7, Settler 8, Mixer 9, Settler 10, Isobutylene Distillation Column 11 and Isoprene Distillation Column 12.

Reactor 1, which is a tubular heat exchanger having 5-liter tubular capacity, an external circulation pipe and a circulation pump designed to maintain liquid phase circulation equal to not less than 500 liters/hour, is fed with 4.8 liters of 6% ortho-phosphoric acid water solution, and, with the circulation pump in operation, hot heat-transferring medium is supplied into the inter-tubular space, and the reactor is heated up to 170°C.

4,4-dimethyl-1,3-dioxane (expenditure: 400.0 g/hour) and 88% trimethyl carbinol (expenditure: 2875.9 g/hour) are fed into the reactor. Pressure maintained in the reactor: 15.0 atmospheres.

Vapor & liquid flow going out of the reactor is throttled, through PC pressure regulator valve, into Separator 2, where pressure is maintained on the level of 7.5 atmospheres. In Separator 2, the flow is separated into a liquid phase and a vapor phase.

Vapors of reaction products and non-transformed source material vapors, having gone out of Separator 2, go through Condensing Refrigerator 7, where they are cooled-down and condensed, and enter Settler 8, where they are split into a water layer and an organic layer.

The flow of liquid products, which is a mixture of ortho-phosphoric acid water solution and high-boiling by-products, are throttled out of Separator 2, through LC-level regulator valve, into Separator 3, where the maintained pressure is 1.5 atmospheres. In Separator 3, the liquid phase achieves its ebullience accompanied with a partial steaming-off of water and organic products. Water vapors and vapors of the products go through Condensing Refrigerator 14, where they are cooled down and condensed, and flow off into Container 5.

The mixture of ortho-phosphoric acid water solution and high-boiling by-products is taken out of Separator 3 and, with use of the pump, delivered, in the quantity of 8.0 l/hour, through Refrigerator 6, into Mixer 9 for extraction of organic products.

As the extracting agent, organic layer of synthesis products is fed into Mixer 9 out of Settler 8. Mixture going out of Mixer 9 enters Settler 10, where it is split into organic layer, containing the extracted high-boiling by-products, and water solution of ortho-phosphoric acid.

Made free of any high-boiling organic products, ortho-phosphoric acid water solution is, with use of the pump, put back into Reactor 1 out of Settler 10.

The organic layer is put out of Settler 10 for separation. Initially, in Rectifying Column 11, isobutylene is isolated in the quantity of 1561.6 g/hour and sent for hydration into trimethyl carbinol (the hydration unit is not shown in the layout illustration); after this, in Rectifying Column 12, isoprene is isolated in the quantity of 359.7 g/hour and delivered for purification.

Column 12 bottom product (in the quantity of 280.3 g/hour), which is a mixture of synthesis by-products and non-transformed source materials, and which contains 67.6% of trimethyl carbinol, 8.2% of methyl dihydropyrane, 2.6% of dimethyl dioxane, 2.4% of isoamylene alcohols and 19.2% of other products, is supplied into equipment unit usable for isolation of non-transformed source materials and interim synthesis products.

Condensed synthesis products' water layer containing 5.9% of trimethyl carbinol, 0.3% of formaldehyde and 0.2% of other organic products is taken (in total, in the quantity of 1074.3 g/hour) out of Settler 8 and Container 5 and sent for purification before discharge into the chemically polluted sewage system (into the unit for distillation of easy-boiling organic products and formaldehyde).

According to the process balance, isoprene output is as follows: 156.2 mole % for dimethyl dioxane, which makes 78.1% of the expected output, and 166.8 mole % for trimethyl carbinol (isobutylene), which makes 83.4% of the expected output.

The process is maintained continuously for 40 hours. Per-substance assay of flows sampled after 8, 20 and 40 hours of operation shows identical results, which evidences absence of any water accumulated in the reactive water layer and stability of parameters of the process.

### Example 6 (for reference)

Layout of equipment for use of the method according to the proposed invention is shown at Fig.6.

The equipment contains Reactor 1, Separator 2, Refrigerator 3, Condensing Refrigerator 4, Settler 5, Mixer 6, Settler 7, Isobutylene Distillation Column 8 and Isoprene Distillation Column 9.

Reactor 1, which is a 5-liter autoclave having a mixer provided with an air-tight electro-magnetic drive and a thermal jacket, is fed with 4.8 liters of 7% ortho-phosphoric acid water solution, and, with the stirring in the ON mode, hot heat-transferring medium is supplied into the thermal jacket, and the reactor is heated up to 160°C.

Supply of the reagents, temperature and pressure maintained in the reaction zone are as shown in Example 1. 40% formaline (expenditure: 256.5 g/hour) and 88% trimethyl carbinol (expenditure: 2875.9 g/hour) are fed into the reactor.

Vapor & liquid flow going out of the reactor enters Separator 2, where it is separated into a liquid phase and a vapor phase.

Reaction product vapors and non-transformed source material vapors, having gone out of Separator 2, proceed to Condensing Refrigerator 4, where they are cooled down and condensed, and enter Settler 5, where they are split into a water layer and an organic layer.

Pressure maintained in the reaction unit (the reactor, the separator, the refrigerator, the settler) is equal to 12 atmospheres.

The flow of liquid products, which is a mixture of ortho-phosphoric acid water solution and high-boiling by-products, are put out of Separator 2 in the quantity of 30.0 l/hour, cooled down in Refrigerator 3 and supplied into Mixer 6 for extraction of organic products. As the extracting agent, the organic layer of synthesis products is supplied into Mixer 6 out of Settler 5. Mixture going out of Mixer 6 enters Settler 7 to be split there into organic layer, containing the extracted high-boiling by-products, and water solution of ortho-phosphoric acid.

Made free of any high-boiling organic products, ortho-phosphoric acid water solution is, with use of the pump, put back into Reactor 1 out of Settler 7.

The organic layer is put out of Settler 7 for separation. Initially, in Rectifying Column 8, isobutylene is isolated and sent for hydration into trimethyl carbinol (the hydration unit is not shown in the layout illustration); after this, in Rectifying Column 9, isoprene is isolated and delivered for purification.

Column 9 bottom product, which is a mixture of synthesis by-products and non-transformed source materials, is delivered into unit usable for isolation of non-transformed source materials and interim synthesis products.

Condensed synthesis products' water layer is taken out of Settled 5 and sent for purification (into unit usable for distillation of high-boiling organic products and formaldehyde) before discharge into the chemically polluted sewage system.

The process is maintained continuously. After 10 hours of operation of the equipment, the volume of catalyzer water solution grows largely (∼ by 1.3 times), while its acidity becomes lower; there is also over-filling of Separator 2 by the liquid phase, its flowing-over into the vapor cooling-down and condensation system and losses of the catalyzer (with the water layer) from Settler 5. The process is stopped.

Attempts at making the process continuous are repeated by way of gradual reduction of pressure in the reaction unit (the reactor, the separator, the settler).

It is under pressure as low as 9.0 atmospheres that it, finally, becomes possible to provide the balance output of water, together with vapors of the products, and to maintain the process continuously (to maintain the liquid phase at a stable level in Separator 2).

According to the process balance, isoprene output, in terms of the transformed source materials, is: 71.8 mole % for formaldehyde and 73.2 mole % for isobutylene.

### Example 7 (for reference)

The process is maintained as shown in Example 6, except that 4,4-dimethyl-1,3-dioxane is supplied instead of formaline. Supply of the reagents, temperature and pressure maintained in the reaction zone are as shown in Example 3.

In case the process is continuous, after 20-hour operation of the equipment, volume of the catalyzer water solution grows largely (by 1.3 times), while its acidity becomes lower; there is also over-filling of Separator 2 by the liquid phase, its flowing-over into vapor cooling-down and condensation system and losses of the catalyzer (with the water layer) from Settler 5. The process is stopped.

Attempts at making the process continuous are repeated by way of gradual increase of temperature in the reactor. It is under temperature as high as 170°C that it finally becomes possible to provide the balance output of water, together with vapors of the products, and to maintain the process continuously (to maintain the liquid phase at a stable level in Separator 2).

According to the process balance, isoprene output is: 144.8 mole % for dimethyl dioxane, which is 72.4% of the expected output, and 149.8 mole % for trimethyl carbinol (isobutylene), which is 74.9% of the expected output.

## Claims

1. Process for the preparation of isoprene by way of liquid-phase interaction between trimethyl carbinol (or its water solutions) and formaldehyde (or its source substances, such as 4,4-dimethyl-1,3-dioxane), in the presence of acidic catalyzer water solution, which can be used under a higher temperature and pressure, in one or several contacting stages, with use of (at the final contacting stage) a separation reactor containing a heat supply zone, a reaction zone and a separation zone, with reaction products and water taken out of the separation zone in the form of a vapor flow (which is followed by the cooling down, condensation and separation), with liquid flow of the catalyzer water solution put out for extraction and, afterwards, returned into heating zone; having the following distinctive feature: reaction zone pressure is maintained higher than saturated water vapors' pressure corresponding to reaction zone temperature, while separation zone temperature is maintained lower than saturated water vapors' pressure corresponding to reaction zone temperature, and while the reactive flow is throttled during its transition from the reaction zone to the separation zone.

2. The process according to claim 1, wherein the reaction zone temperature is maintained at the level of 140-180 °C, with pressure equal to 8-25 atmospheres, while pressure maintained at the separation zone is 1.2-9,5 atmospheres.

3. The process according to claim 1 or 2, wherein the usable separation reactor contains two successive separation zones: flow going out of the reaction zone is throttled into Separation Zone 1 (with pressure reduced by 2-20 atmospheres), wherefrom the vapor flow is sent for cooling down and condensation, while the liquid flow is throttled into Separation Zone 2 (with pressure reduced down to 1.2-2.0 atmospheres), and this is accompanied with taking out, cooling down and condensation of the vapor phase and taking out, cooling down, extraction and re-cycling of the liquid phase into the heating zone.

4. The process according to claim 1 or 2, wherein the usable separation reactor contains three separation zones: Zone 1 for preliminary separation under temperature and pressure levels closely similar to those in the reaction zone; a flow containing, predominantly, vapor (gaseous) phase is taken out of the upper part of Separation Zone 1 and throttled into Separation Zone 2 (with pressure reduced by 2-20 atmospheres); flows containing, predominantly, liquid water solution of the catalyzer are taken out of the lower part of Separation Zones 1 and 2 and throttled into Separation Zone 3 (with pressure reduced down to 1.2-2.0 atmospheres), with the vapor phase taken out, cooled down and condensed and with the liquid phase taken out, cooled down, extracted and re-cycled into the heating zone.

5. The process according to claim 1 or 2, wherein circulation of the reactive water phase along the following circuit: the reaction zone - the separation zone - the extraction zone - the heating zone - the reaction zone is performed in the quantity of 0.2-6.0 parts of the total reaction zone area.
